# EUROPEAN PATENT SPECIFICATION

(11) **EP 1 301 537 B1**
(45) Date of publication and mention of the grant of the patent: **17.09.2008**
(21) Application number: 01949734.6
(22) Date of filing: 18.07.2001
(51) Int. Cl.: C07K 14/775, G01N 33/92

(54) **PEPTIDES AND THEIR USE IN ASSAYS FOR CARDIOVASCULAR DISEASE**
PEPTIDE UND IHRE VERWENDUNG IN DER DIAGNOSTIK VON KARDIOVASKULÄREN ERKRANKUNGEN
UTILISATION DE PEPTIDES DANS DES DOSAGES POUR MALADIE CARDIO-VASCULAIRE

(30) Priority: 18.07.2000 GB 0017641
(43) Date of publication of application: 16.04.2003
(73) Proprietor: Ark Therapeutics Oy, 70210 Kuopio (FI)
(72) Inventor: NARVANEN, Outi, c/o Ark Therapeutics Oy, FIN-70210 Kuopio (FI); YLA-HERTTUALA, Seppo, c/o A.I. Virtanen Institute, FIN-70211 Kuopio (FI)
(74) Representative: Perry, Robert Edward
(86) International application number: PCT/GB2001/003212
(87) International publication number: WO 2002/006314

(56) References cited:
- WO-A-97/43311
- WO-A-98/42751
- WO-A-98/56938
- WO-A-98/59248
- US-A- 5 408 038
- VIITA H ET AL.: "Different apolipoprotein B breakdown patterns in models of oxidized low density lipoprotein" LIFE SCIENCES, vol. 65, no. 8, 1999, pages 783-793, XP001053293
- CHEN PF ET AL.: "Primary sequence mapping of human apolipoprotein B-100 epitopes" EUROPEAN JOURNAL OF BIOCHEMISTRY, vol. 175, 15 July 1988 (1988-07-15), pages 111-118, XP001053294
- MIRONOVA M ET AL.: "Isolation and Characterization of Human Antioxidized LDL Antibodies" ARTERIOSCLEROSIS, THROMBOSIS, AND VASCULAR BIOLOGY, vol. 16, no. 2, 1996, pages 222-229,

## Description

### Field of the Invention

This invention relates to the preparation of peptides for use in assays for evaluating the risk of coronary heart diseases and other cardiovascular diseases.

### Background to the Invention

Coronary heart disease (CHD) is the leading cause of death in European countries. In most cases, the basic cause of CHD is atherosclerosis. Currently, the risk of atherosclerosis is evaluated by measuring the amount of total cholesterol, low density lipoprotein (LDL) and high density lipoprotein (HDL). However, these tests do not predict the disease in approximately one third of the patients; see Yla-Herttuala, Current Opinion Lipidol. (1998) 9: 337-344. There is therefore a need to develop a better assay to predict the risk for CHD.

Oxidized low density lipoprotein (oxLDL) has been shown to be a risk factor in atherosclerosis, but it has not been possible to measure oxLDL directly in plasma because its half-life in circulation is short. Recent studies have therefore focused on different indirect measurements to define the extent of LDL oxidation.

OxLDL plays an important role in atherogenesis. It has been detected in atherosclerotic lesions, is cytotoxic to various cell types and chemotactic for blood monocytes. In addition, oxLDL is immunogenic, and atherosclerotic lesions contain immunoglobulins that recognize oxLDL; autoantibodies against oxLDL are present in human and rabbit sera. The best way to analyze oxLDL appears to be the measurement of autoantibodies against oxLDL, as suggested by Yla-Herttuala, *supra.*

Apolipoprotein B-100 (apoB-100) is the major protein constituent in LDL. The human cDNA and amino-acid sequences are reported by Chen et al., J. Biol. Chem. (1986) 261: 12912-12921.

During oxidation of LDL, both the protein and the lipid portion of the particle can be modified. Malondialdehyde (MDA) and 4-hydroxynonenal (4-HNE) are the main reactive aldehydes formed during LDL oxidation, as reported by Esterbauer et al., Free Radical Biol. Med. (1992) 13:341-390 or by Viita et al., Life Sciences (1999) 65: 783-793 each can further react with lysine residues of apoB-100. These poorly characterized oxidation specific epitopes are recognized by the autoantibodies. More recently, it has been suggested by Palinski et al., J. Clin. Invest. (1996) 98: 800-814, that oxidized phospholipids are epitopes for autoantibodies. In addition, it has been reported that healthy individuals produce antibodies against lysophosphatidylcholine, which is a major component of oxLDL.

Anti-oxLDL autoantibodies may predict progression of carotid atherosclerosis, coronary atherosclerosis and myocardial infarction. Elevated levels of autoantibodies have also been found in systemic lupus erythematosus (SLE), pre-eclampsia, chronic periaortitis, non-insulin-dependent diabetes mellitus and in endothelial dysfunction. Mironova et al., Arteriosclerosis, Thombosis, and Vascular Biology (1996) 16 : 222-229, report a competitive EIA wherein serum containing Ox-LDL antibodies is contacted with immobilized Ox-LDL.

Autoantibodies against oxLDL nave been measured using very different immunoassays (EIA or RIA), and no standard method or reference material is available for the standardization of the assays. LDL used in previous tests has been purified from human plasma and is usually oxidized by incubation with copper ions or by conjugation with MDA. Copper-oxidized LDL contains a mixture of oxidation-specific epitopes, and therefore the oxidation process must be standardized carefully to produce homogeneous antigen. Human plasma LDL-based antigens are also inherently unstable and are not suitable for the production of commercial test kits. Therefore, there is a need to produce an assay for CHD that can be standardised and which makes use of reagents which are stable and give reproducible results.

### Summary of the Invention

The present invention is based on the realisation that peptides which consist of 20 to 40 amino acids and which comprise SEQ ID NO: 2 are stable and can be used as antigens in an immunoassay for CHD, and have affinity for oxidised low density lipoprotein.

According to one aspect of the invention, a peptide of the invention is used in an immunoassay to determine the presence and, optionally, the amount of antibodies, in a sample, having affinity for oxidised low density lipoprotein.

According to a second aspect, a method for measuring the amount of autoantibodies for oxidised low density lipoprotein in a sample, comprises:
(i) contacting the sample with immobilised peptides as defined above, under conditions which permit the autoantibodies to bind to the peptides; and
(ii) determining the amount of binding.

The amount of binding can be measured directly and will correlate to the amount of oxidised LDL in a sample. The amount of antibodies can be expressed as the ratio of antibody binding between oxidised LDL and native LDL.

The use of the peptides of the present invention ensures that the immunoassay uses a stable antigen which provides reproducible results.

According to a third aspect, a kit for measuring autoantibodies of oxidised LDL, comprises a multicontainer unit having:
(i) a composition comprising peptides as defined above; and
(ii) reagents necessary to carry out an immunoabsorption assay.

The present invention provides reagents that can be synthesised easily without the need to isolate proteins from a patient's blood. The peptides do not have the short half-life associated with the proteins used in conventional assays for CHD and therefore can be manufactured commercially for use in diagnostic kits.

### Description of the Invention

The present invention relies on the production of peptides which are preferably derived from apoB-100 protein, or which preferably have an amino acid sequence which forms a structure similar to that of the epitopes on apoB-100 protein. The peptides are therefore able to undergo specific interaction with autoantibodies which have affinity for oxidised LDL. The term "specific interaction" refers to the recognition of the autoantibodies for the peptide (antigen). The peptides may elicit antibody binding with an affinity constant of greater than 10⁵ l/mol, preferably greater than 10⁷ l/mol and more preferably greater than 10⁸ l/mol.

In principle, any peptide sequence of approximately greater than 20 amino acids may be used in the present invention provided that it acts as a ligand for the autoantibodies. The peptide may be derived from a natural source of apoB-100 or may be a synthetic peptide based on the known protein sequence for apoB-100. Methods to isolate peptides from apoB-100 or to synthesis peptides, will be apparent to the skilled person.

The size of the peptides is sufficient for recognition by the autoantibodies. The peptides of the present invention are 20-40 amino acids in size, preferably 20-30 amino acids and comprise SEQ ID NO: 2.

The peptides of the present invention may be used in a diagnostic assay together with other reagents capable of eliciting an antibody reaction. For example, phosphatidyl ethanolamine can be derivatised with MDA and, when used with the peptides of the invention, is capable of acting as an epitope for some autoantibodies.

The new peptide-based EIA assay could be used as a test kit for the evaluation and follow-up of patients with cardiovascular diseases and several other disorders, such as periaortitis, pre-eclampsia, non-insulin-dependent diabetes and endothelial dysfunction.

When used in the immunoassay, it is preferable that the peptides are immobilised on a solid support, as this enables subsequent washing steps to be carried out easily. Methods to carry out immunoassays will be apparent to the skilled person.

The following Examples illustrate the invention.

### Example 1

Various native and modified peptides, derived from the amino acid sequence of apoB-100 (Chen *et al., supra*; WO 97/43311), were tested as antigens suitable for use in EIA. The peptides were modified with MDA to produce similar oxidation specific epitopes as in oxLDL. The results of peptide EIA are compared to the results of oxLDL EIA which was optimized using copper-oxidized LDL as antigen; see Närvänen et al., Free Radical Biology & Medicine (2001) 31:769-777.

Peptides were synthesized by using solid-phase peptide synthesis technology and Fmoc chemistry and purified with HPLC using a C₁₈ column. The molecular weights of the synthesized peptides were identified by using a MALDI-TOF mass spectrometer. Peptide sequences either derived from or unrelated to the amino acid sequence of apoB-100 are shown in Table 1.

**Table 1. Sequences of the synthetic peptides**

| Peptide | Sequence | Number of amino acids | Number of lysines (=K) |
|---|---|---|---|
| p62 SEQ* ID NO. 1 | DIVAHLLSSSSSVIDALQYKLEGTTRLTRKRGLK | 35 | 3 |
| p63 SEQ ID NO. 2 | LSVKAQYKKNKHRHSITNPL | 20 | 4 |
| p64 SEQ* ID NO 3. | STTVMNPYMKLAPGELTIIL | 20 | 1 |
| p65 SEQ* ID NO. 4 | KKKKKKKKKKKKKKKKKKKKKKKKKKKKKKKKKKK | 35 | 35 |

| | | | |
|---|---|---|---|
| * these peptides are listed for illustration only. | | | |

### Modification of the peptide with MDA

MDA was coupled to the peptide via the primary amines of lysines by using a modification of the method of Palinski et al., Arteriosclerosis (1990) 10: 325-335. MDA was made freshly from malondialdehyde-bis(dimethyl acetal) by acid hydrolysis. 10 moles of MDA was then coupled to 1 mole of peptide by stirring the mixture for 3 hours at 37°C. The efficiency of coupling was checked by ninhydrin reaction which reveals the presence of primary amines.

Serum and plasma samples for peptide EIA tests were collected from ongoing studies including samples from patients with suspected coronary heart disease and from healthy controls. These samples were stored in aliquots at -20°C.

### Autoantibodies against peptides and peptide-MDA complexes (peptide EIA)

Native and MDA-modified peptides were tested always on the same plate. One half of the plate was coated with the native peptide (20 µg/ml) and the other half was coated with the MDA-modified peptide [(20 µg/ml) 100 µl/well in 100 mmol/l bicarbonate buffer (pH 9.5)]. Coated plates were incubated overnight at room temperature (RT) and then washed with an automatic washer (Wellwash 4 MK II, Labsystems Oy) three times with phosphate buffered saline (PBS) containing 0.05% Tween 20. Plates were blocked with PBS containing 1% human serum albumin (HSA) (150 µl/well) for 1 h at RT and washed as above. Serum samples were diluted 1:20 in PBS containing 0.2% HSA and 0.05% Tween 20 and pipetted 100 µl/well. Plates were incubated for 2 h at RT and washed as above. HRP-conjugated anti-human IgG (diluted 1:20 000 in the sample buffer) was added (100 µl) to each well and incubated for 1 h at RT. After washing, colour was developed by adding the peroxidase substrate (tetramethylbenzidine (TMB) as a chromagen, 100 µl/well) and incubating the plates for 30 min at room temperature in the dark. The reaction was stopped with 0.5 mol/l H₂SO₄ (100 µl/well) and absorbances were measured at 450 nm (Multiskan microplate reader, Labsystems Oy). The results are shown in Table 2 and are expressed as the absorbance measurement obtained for the native and the modified peptides or as the ratio between antibody binding to the native and the modified peptides after subtracting the blank control.

**Table 2: Reactivity of autoantibodies**

| A. Healthy controls (n=17). | | | |
|---|---|---|---|
| | OxLDL EIA | | PEPTIDE EIA |
| oxLDL:natLDL ratio | 1.886 ± 0.689 | MDAp63:natp63 ratio | 2.592 ± 0.751 |
| oxLDL | 0.591 ± 0.221 | MDAp63 | 0.497 ± 0.184 |
| natLDL | 0.351 ± 0.172 | natp63 | 0.216 ± 0.142 |

| B. Patients (n=19). | | | |
|---|---|---|---|
| | OxLDL EIA | | PEPTIDE EIA |
| oxLDL:natLDL ratio | 2.535 ± 1.430 | MDAp63:natp63 ratio | 2.866 ± 0.924 |
| oxLDL | 0.600 ± 0.287 | MDAp63 | 0.418 ± 0.141 |
| natLDL | 0.258 ± 0.170 | natp63 | 0.159 ± 0.079 |

Based on the ratio values, the amount of autoantibodies was higher in patient samples than in controls when tested with the peptide EIA and the human LDL-based oxLDL EIA. The reactions of autoantibodies using the native and modified (peptide) antigens are similar in oxLDL-EIA and peptide-EIA because the modified antigens (oxLDL and MDAp63) are recognized better than the native antigens (natLDL and natp63), i.e. there is greater specificity for the modified antigens. The results for the peptide-EIA are significantly better than for the oxLDL-EIA.

To find out whether autoantibodies react with the peptide, with MDA or both when peptide-MDA complexes are used as antigen, a microtiter plate was coated with natp63, MDA and MDAp63 antigen and the immunoassays performed. MDA antigen was prepared as MDAp63 antigen but without peptide and MDA was diluted for coating in the same way as MDAp63. The reaction of autoantibodies was higher with MDAp63 than with natp63 and MDA, in both control (0.533±0.077, 0.282±0.100 and 0.300±0.019, respectively, mean±SD n=23) and patient samples (0.431±0.040, 0.222±0.041 and 0.338±0.000, respectively, n=16), indicating that both peptide and MDA are needed for optimal recognition.

Spearman correlation coefficients were calculated between antibodies against natp63, MDA and MDAp63. Anti-MDA and anti-MDAp63 antibodies correlated in control and patient samples (r=0.581 p=0.004 and r=0.582 p=0.018, respectively), but anti-natp63 and anti-MDA antibodies did not correlate (r=0.297 p=0.169 and r=0.112 p=0.68, respectively). This shows that different antibodies react with MDA and natp63 and is evidence that the peptide-MDA complex is the optimal antigen.

### Example 2

Six different forms of peptide p63 were tested, to find out what is the best modification of the peptide for EIA:
linear p63 (p63)
p63 modified with MDA (p63-MDA)*.
p63 modified with MDA and phosphatidylethanolamine (p63-PEA-MDA)*
a linear form of p63, which contains two extra amino acids (glycine and cysteine)
at both ends of the peptide (p244); see SEQ ID NO. 5
cyclic p244 (p244cyc)*
cynic p63 modified with MDA (p244cyc-MDA)*
* these modified peptides are cited for comparison only.

These modified peptides are cited for comparison only. The amount of autoantibodies was highest in patient samples when the linear peptide p244 was used as antigen (Table 3). Antibody titers against cyclic peptide 244cyc were also slightly higher than against p63-MDA. In this study autoantibodies reacted most with peptides without lipids.

### Specificity studies

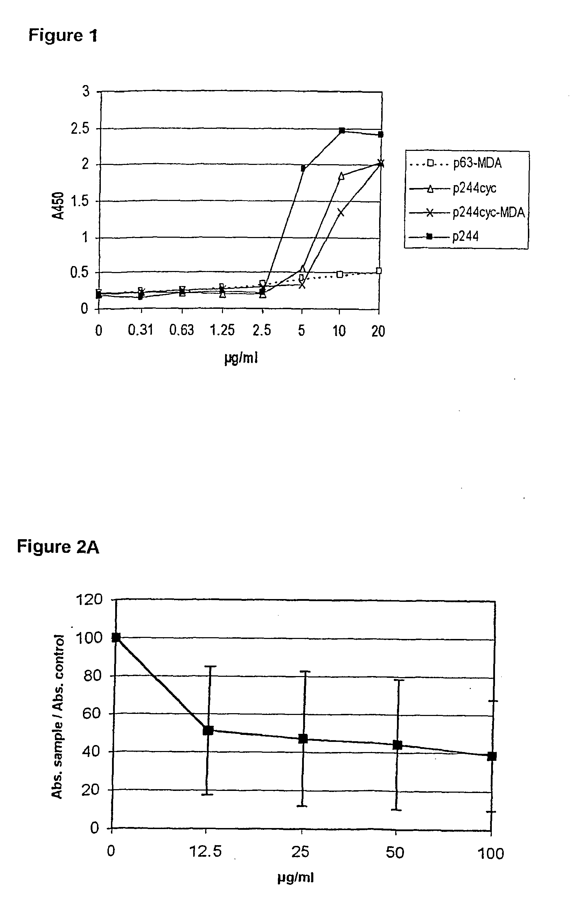

The specificity of different antigens was tested by titration of peptides. Figure 1 shows titration curves for 4 peptide antigens tested with one patient sample. In addition, the specificity of antibodies against cyclic and linear peptides, p244cyc and p244, was tested by adding 0.5 M sodium chloride to sample diluent. The titer of patient samples against p244, p244cyc and p63-MDA was 19%, 38% and 47% lower (mean of 20 patient samples), respectively, when salt was added to sample diluent. It appears that antibodies against p244 have the best specificity.

### Inhibition studies

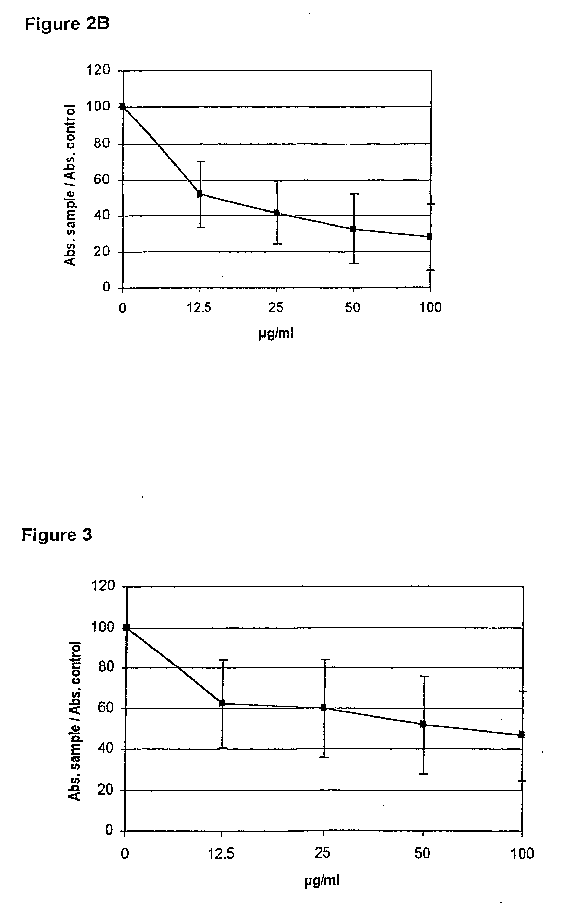

The specificity of cyclic (p244cyc) and the corresponding linear peptide (p244) were tested in inhibition tests by adding increasing concentrations of peptides to sample diluent. P244cyc peptide inhibited with the highest inhibitor concentration (100 µg/ml), the average being 61% of the binding of 8 patient samples (Fig. 2A); 3 samples were inhibited by less than 50%. P244 inhibited with the highest inhibitor concentration (100 µg/ml), the average being 70% of the binding of 24 patient samples (Fig. 2B). 3 samples were inhibited less than 50%. P244 seemed to be more specific than p244cyc peptide. Finally, it was confirmed that the epitope of p244 is similar to p63 by coating plate with p244 and inhibiting the binding of patient samples with p63. P63 inhibited with the highest inhibitor concentration (100 µg/ml), the average being 53% of the binding of 22 patient samples (Fig. 3). 9 samples were inhibited less than 50%.

### Modification of p244

As a summary of previous results it appears that patient samples are best recognized by the peptide p244. Therefore, modified p244 was modified with MDA and p244 and p244-MDA were both used as antigen in EIA. The titers of patient samples (mean ± SD) against p244 and p244-MDA were slightly higher than titers of control samples (Table 5).

### Evaluation of peptide EIA

205 samples of patients with symptoms of CHD were analysed. The patients were angiographically tested and classified as 0-, 1-, 2-, or 3-vessel disease. Peptides p244 and p244-MDA were used as antigen in peptide EIA, and the results compared to human-LDL-based oxLDL EIA.

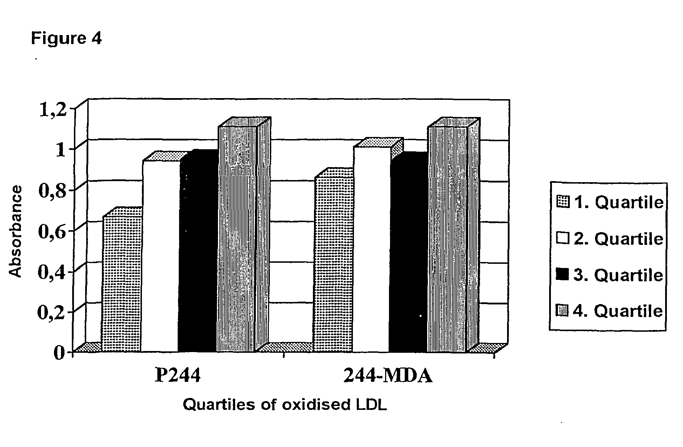

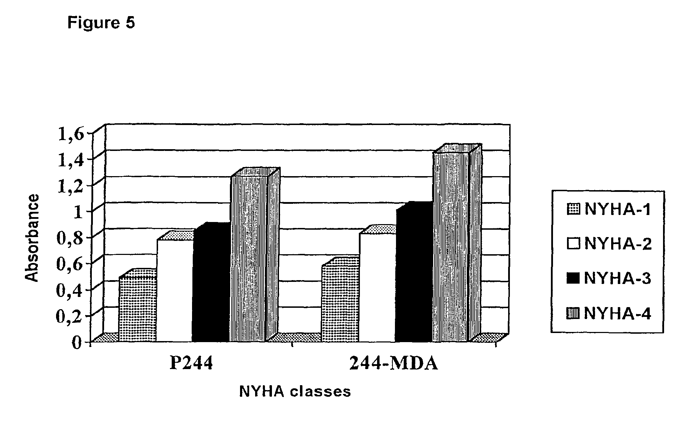

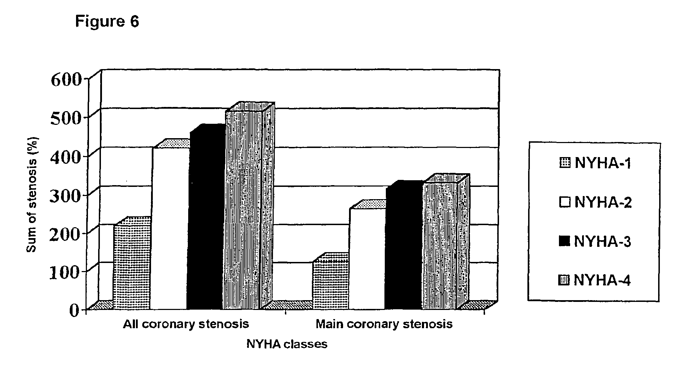

The titers of autoantibodies against p244-MDA were highest in those patients with 3-vessel disease compared to 0-, 1-, or 2-vessel disease (ANOVA p=0.0268). The titers of autoantibodies against human-LDL-based oxLDL (n=185) correlated with the titers against p244 (r=0.227 p=0.0019) and with the titers against p244-MDA (p=0.217 p=0.003). The correlation was confirmed when the titers of autoantibodies against peptides p244 and p244-MDA were compared to the titers of autoantibodies against human-LDL-based oxLDL classified tofourgroups based on anti-oxLDL titer (1. quartile 0.072-0.22 (n=37), 2. quartile 0.22-0.36 (n=44), 3. quartile 0.36-0.63 (n=57), and 4. quartile 0.63-2.757 (n=68)) (Fig. 4). In addition, we compared the amount of antibodies against p244 and p244-MDA to angina pectoris symptoms classified according to the New York Heart Association (NYHA) heart disease classification (NYHA-1 n=5, NYHA-2 n=12, NYHA-3 n=27, and NYHA-4 n=14). We found that antibody titers increased when angina pectoris symptoms increased (Fig. 5). The total sum of coronary stenosis compared with NYHA classification (NYHA-1 n=14, NYHA-2 n=26, NYHA-3 n=57, and NYHA-4 n=29) showed more serious symptoms with the progression of coronary disease (Fig. 6). This result revealed that the total sum of coronary stenosis examined using coronary angiography is correlated as well as the antibodies with the seriousness of angina pectoris symptoms.

**Table 3. The amount of autoantibodies against six different forms of p63 peptide antigen**

| Peptide antigen | P63 | P63-MDA* | P63-PEA-MDA* | P244cyc* | P244cyc-MDA* | P244 |
|---|---|---|---|---|---|---|
| Patients (n=20) | 0.389 ± 0.353 | 0.601 ± 0.192 | 0.406 ± 0.215 | 0.734 ± 0.520 | 0.521 ± 0.504 | 0.995 ± 0.570 |

| | | | | | | |
|---|---|---|---|---|---|---|
| * these peptide derivatives are cited for comparison only | | | | | | |

Values are mean absorbances ± SD after subtracting the blank.

**Table 4. The amount of autoantibodies against p244 and MDA modified p244 peptide**

| Peptide antigen | P244 | P244-MDA* |
|---|---|---|
| Controls (n=23) | 0.870 ± 0.371 | 0.956 ± 0.307 |
| Patients (n=20) | 1.074 ± 0.610 | 1.296 ± 0.544 |

| | | |
|---|---|---|
| * this modified peptide is cited for comparison only | | |

Values are mean absorbances ± SD after subtracting the blank.

## Claims

1. A peptide which consists of 20 to 40 amino acids and which comprises SEQ ID NO.2.

2. Use of a peptide according to claim 1 in an immunoassay, to determine the presence and, optionally, the amount of autoantibodies in a sample.

3. Use according to claim 2, wherein the peptide interacts with the autoantibodies with an affinity constant of greater than 10⁵ l/mol.

4. A method for measuring the amount of autoantibodies for oxidised low density lipoprotein in a sample, comprising:
(i) contacting the sample with an immobilised peptide according to claim 1, under conditions which permit the autoantibodies to bind to the peptides; and
(ii) determining the amount of binding.

5. A method according to claim 4, wherein the sample is a serum or plasma sample from a patient.

6. A kit suitable for measuring autoantibodies of oxidised LDL, comprising a unit including containers respectively containing:
(i) a peptide according to claim 1; and
(ii) reagents necessary to carry out an immunoadsorbence assay.

## Patentansprüche

1. Peptid, das aus 20 bis 40 Aminosäuren besteht und welches SEQ ID NO. 2 umfasst.

2. Verwendung eines Peptids nach Anspruch 1 in einem Immunoassay, um das Vorhandensein und gegebenenfalls die Menge von Autoantikörpern in einer Probe zu bestimmen.

3. Verwendung nach Anspruch 2, wobei das Peptid mit den Autoantikörpern mit einer Affinitätskonstante höher als 10⁵ l/mol wechselwirkt.

4. Verfahren zum Messen der Menge von Autoantikörpern gegen oxidiertes Lipoprotein geringer Dichte in einer Probe, umfassend:
(i) Inkontaktbringen der Probe mit einem immobilisierten Peptid nach Anspruch 1 unter Bedingungen, welche es den Autoantikörpern erlauben, an die Peptide zu binden; und
(ii) Bestimmen der Menge an Bindung.

5. Verfahren nach Anspruch 4, wobei die Probe eine Serum- oder Plasmaprobe von einem Patienten ist.

6. Kit, welcher zum Messen von Autoantikörpern gegen oxidiertes LDL geeignet ist, umfassend eine Einheit, welche Behälter umfasst, welche jeweils enthalten:
(i) ein Peptid nach Anspruch 1; und
(ii) Reagenzien, die notwendig sind, um einen Immunadsorptionsassay auszuführen.

## Revendications

1. Peptide formé de 20 à 40 acides aminés, et qui comprend SEQ ID NO. 2.

2. Utilisation d'un peptide selon la revendication 1 dans un test immunologique, pour déterminer la présence et, facultativement, la quantité d'auto-anticorps dans un échantillon.

3. Utilisation selon la revendication 2, dans laquelle le peptide interagit avec les auto-anticorps avec une constante d'affinité supérieure à 10⁵ l/mol.

4. Procédé pour mesurer la quantité d'auto-anticorps dirigés contre une lipoprotéine de faible densité, oxydée, dans un échantillon, comprenant les étapes consistant à:
(i) mettre l'échantillon en contact avec un peptide immobilisé selon la revendication 1, dans des conditions qui permettent aux auto-anticorps de se lier aux peptides; et
(ii) déterminer l'ampleur de la liaison

5. Procédé selon la revendication 4, dans lequel l'échantillon est un échantillon de sérum ou de plasma provenant d'un patient.

6. Trousse convenant pour mesurer des auto-anticorps anti LDL oxydée, comprenant un ensemble incluant des récipients contenant, respectivement :
(i) un peptide selon la revendication 1, et
(ii) des réactifs nécessaires pour effectuer un test d'immunoabsorption.


### SEQUENCE LISTING

<110> Ark Therapeutics Ltd.
<120> PEPTIDES AND THEIR USE IN ASSAYS FOR CARDIOVASCULAR DISEASE
<130> REP06363WO
<140> not yet known
   <141> 2001-07-18
<160> 5
<170> PatentIn Ver. 2.1
<210> 1
   <211> 34
   <212> PRT
   <213> Artificial Sequence
<220>
   <223> Description of Artificial Sequence: oligopeptide
<400> 1
   Asp Ile Val Ala His Leu Leu Ser Ser Ser Ser Ser Val Ile Asp Ala
    1               5                  10                  15
   Leu Gln Tyr Lys Leu Glu Gly Thr Thr Arg Leu Thr Arg Lys Arg Gly
               20                  25                  30
   Leu Lys
<210> 2
   <211> 20
   <212> PRT
   <213> Artificial Sequence
<220>
   <223> Description of Artificial Sequence: oligopeptide
<400> 2
   Leu Ser Val Lys Ala Gln Tyr Lys Lys Asn Lys His Arg His Ser Ile
    1               5                  10                  15
   Thr Asn Pro Leu
               20
<210> 3
   <211> 20
   <212> PRT
   <213> Artificial Sequence
<220>
   <223> Description of Artificial Sequence: oligopeptide
<400> 3
   Ser Thr Thr Val Met Asn Pro Tyr Met Lys Leu Ala Pro Gly Glu Leu
    1               5                  10                  15
   Thr Ile Ile Leu
               20
<210> 4
   <211> 35
   <212> PRT
   <213> Artificial Sequence
<220>
   <223> Description of Artificial Sequence: oligopeptide
<400> 4
   Lys Lys Lys Lys Lys Lys Lys Lys Lys Lys Lys Lys Lys Lys Lys Lys
    1               5                  10                  15
   Lys Lys Lys Lys Lys Lys Lys Lys Lys Lys Lys Lys Lys Lys Lys Lys
               20                  25                  30
   Lys Lys Lys
           35
<210> 5
   <211> 24
   <212> PRT
   <213> Artificial Sequence
<220>
   <223> Description of Artificial Sequence: oligopeptide
<400> 5
   Cys Gly Leu Ser Val Lys Ala Gln Tyr Lys Lys Asn Lys His Arg His
    1               5                  10                  15
   Ser Ile Thr Asn Pro Leu Gly Cys
               20